# EUROPEAN PATENT APPLICATION

(11) **EP 4 484 545 A1**
(43) Date of publication of application: **01.01.2025**
(21) Application number: 23756200.4
(22) Date of filing: 03.02.2023
(51) Int. Cl.: C12M 1/42, C12N 15/87

(54) **ELECTROPORATION DEVICE**

(30) Priority: 21.02.2022 JP 2022024662
(71) Applicant: FUJIFILM Corporation, Tokyo 106-8620 (JP)
(72) Inventor: YAKUSHIJI, Takashi, Ashigarakami-gun, Kanagawa 258-8577 (JP)
(74) Representative: Hoffmann Eitle
(86) International application number: PCT/JP2023/003572
(87) International publication number: WO 2023/157673

(57) **Abstract**

An object of the present invention is to provide an electroporation device in which an introduction efficiency is unlikely to decrease. An electroporation device according to the embodiment of the present invention applies an electric field to a suspension containing a biologically derived material and a bioactive substance using an electrode pair to introduce the bioactive substance into the biologically derived material, and each of electrodes of the electrode pair has a boron-doped diamond membrane on a surface portion in contact with the suspension.

## Description

### BACKGROUND OF THE INVENTION

### 1. Field of the Invention

The present invention relates to an electroporation device.

### 2. Description of the Related Art

An electric perforating method is a method of introducing a substance into cells by perforating holes in a cell membrane with an electric pulse and is also called electroporation. For example, fine holes can be made in a cell membrane by applying an electric field to a cell suspension using an electrode pair, and cells can be transformed by introducing deoxyribonucleic acid (DNA) into the cells.

A batch type is widely used as electroporation for introducing a bioactive substance such as DNA, ribonucleic acid (RNA), and protein into a biologically derived material such as a cell, a cell derivative, a cell organelle, an intracellular granule, and a vesicle by using an electric perforating method. In the batch type, for example, a suspension containing a biologically derived material and a bioactive substance is accommodated in a container having an inner surface with an electrode pair installed thereon, and an electric field is applied by the electrode pair. As a result, fine holes are created in a membrane covering a surface of the biologically derived material, and the permeability of the membrane is increased. Here, the bioactive substance passes through the membrane with increased permeability by diffusion or electrophoresis, and thus the bioactive substance is introduced into the biologically derived material.

Meanwhile, flow electroporation has also been developed in which a suspension containing a biologically derived material and a bioactive substance is allowed to flow in a flow channel in which an electrode pair is installed (for example, JP2007-7430A). The flow type is suitable for processing a large amount of suspension.

### SUMMARY OF THE INVENTION

In electroporation, a biologically derived material such as a cell may be damaged due to the application of an electric field, heat generation of a suspension, poisoning by a material constituting an electroporation device, or the like. Such damage leads to the death of the biologically derived material or the bioactive substance, and as a result, may lead to a decrease in introduction efficiency (the percentage of the bioactive substance introduced into the biologically derived material subjected to electroporation).

The present inventors have conducted studies on batch electroporation devices and flow electroporation devices of the related art, and found that the introduction efficiency may gradually decrease in a case where the electroporation device is repeatedly used. In particular, it has been found that the problem is remarkable in flow electroporation for processing a large amount of suspension.

In consideration of the above-described circumstances, an object of the present invention is to provide an electroporation device in which the introduction efficiency is unlikely to decrease.

The present inventors have conducted intensive studies to solve the problem. Specifically, the present inventors have performed flow electroporation and repeated batch electroporation using various electrode pairs, and examined temporal changes of the survival rate of a biologically derived material such as a cell and the introduction efficiency. As a result, while finding various electrodes that are less likely to cause a decrease in survival rate, such as an electrode (Pt membrane electrode) on which platinum (Pt) is plated and an electrode (Au membrane electrode) on which gold (Au) is plated, the present inventors have found that in a case where an electrode having a boron-doped diamond membrane at a specific site is used, not only is the survival rate less likely to decrease, but the introduction efficiency is also less likely to specifically decrease.

That is, the present inventors have found that the above-described object can be achieved by the following configurations.
(1) An electroporation device that applies an electric field to a suspension containing a biologically derived material and a bioactive substance using an electrode pair to introduce the bioactive substance into the biologically derived material,
   in which each of electrodes of the electrode pair has a boron-doped diamond membrane on a surface portion in contact with the suspension.
(2) The electroporation device according to (1), in which a boron content of the boron-doped diamond membrane is 100 to 10,000 ppm.
(3) The electroporation device according to (1) or (2), in which a resistivity of the boron-doped diamond membrane is 1 × 10⁻³ to 1 × 10⁻¹ Ω·cm.
(4) The electroporation device according to any one of (1) to (3), in which a membrane thickness of the boron-doped diamond membrane is 2 to 20 µm.
(5) The electroporation device according to any one of (1) to (4), in which an inter-electrode distance of the electrode pair is 1 to 10 mm.
(6) The electroporation device according to any one of (1) to (5), in which each of the electrodes includes a support and the boron-doped diamond membrane covering the support.
(7) The electroporation device according to (6), in which a thickness of the support is 0.5 to 10 mm.
(8) The electroporation device according to (6) or (7), in which the support is a metal substrate.
(9) The electroporation device according to (8), in which a metal material of the metal substrate includes at least one selected from the group consisting of Mo, Nb, W, and Ti.
(10) The electroporation device according to any one of (1) to (9), in which the boron-doped diamond membrane is provided not only on the surface portion in contact with the suspension, but also on a portion that is equal to or more than half or 0.5 mm or more of a side surface portion of the electrode in a thickness direction or on an entire surface of the side surface portion continuously from the surface portion.
(11) The electroporation device according to any one of (1) to (10), in which a cooling mechanism that cools each of the electrodes is provided.
(12) The electroporation device according to any one (1) to (11) that is used for repeated batch electroporation,
   in which a container for accommodating the suspension is provided, and
   the electrode pair is installed on an inner surface of the container so that the boron-doped diamond membranes of the electrodes face each other.
(13) The electroporation device according to any one of (1) to (11) that is used for flow electroporation,
   in which a flow channel for allowing the suspension to flow is provided, and
   the electrode pair is installed with the flow channel interposed therebetween so that the boron-doped diamond membranes of the electrodes face each other.

As shown below, according to the present invention, it is possible to provide an electroporation device in which the introduction efficiency is unlikely to decrease.

### BRIEF DESCRIPTION OF THE DRAWINGS

Fig. 1A is a top view showing an example of an electroporation device according to a first embodiment.
Fig. 1B is a cross-sectional view taken along the line 1B-1B in Fig. 1A.
Fig. 1C is a cross-sectional view of an upper electrode 41a and a lower electrode 41b.
Fig. 1D is a top view showing a modification example of the electroporation device according to the first embodiment.
Fig. 1E is a cross-sectional view taken along the line 1E-1E in Fig. 1D.
Fig. 2 is a cross-sectional view of an upper electrode 42a and a lower electrode 42b in an example of electroporation according to a second embodiment.
Fig. 3 is a cross-sectional view of an upper electrode 43a and a lower electrode 43b in an example of electroporation according to a third embodiment.
Fig. 4 is a cross-sectional view of an upper electrode 44a and a lower electrode 44b in an example of electroporation according to a fourth embodiment.
Fig. 5 is a cross-sectional view showing an example of an electroporation device according to a fifth embodiment.

### DESCRIPTION OF THE PREFERRED EMBODIMENTS

Hereinafter, embodiments of an electroporation device according to the embodiment of the present invention will be described.

Here, first to fifth embodiments correspond to a device used for flow electroporation, and a sixth embodiment corresponds to a device used for batch electroporation. The electroporation device according to the embodiment of the present invention is particularly useful for flow electroporation for processing a large amount of suspension since the introduction efficiency is unlikely to decrease.

In addition, in the present specification, a numerical range expressed using "to" means a range including numerical values described before and after the preposition "to" as a lower limit value and an upper limit value.

In addition, the "suspension containing a biologically derived material and a bioactive substance" is also simply referred to as "suspension", the "boron-doped diamond membrane" is also referred to as "BDD membrane", the "surface portion of an electrode in contact with a suspension" is also referred to as "liquid contact portion", the "electrode having a BDD membrane on a liquid contact portion" is also referred to as "BDD membrane electrode", the "electroporation" is also referred to as "EP", and the "electroporation device" is also referred to as "EP device".

In addition, the fact that the introduction efficiency is unlikely to decrease and the initial introduction efficiency is high is collectively referred to as "the effects and the like of the present invention are excellent".

### [First Embodiment]

An electroporation device according to a first embodiment of the present invention is
an electroporation device that is used for flow electroporation in which an electric field is applied to a suspension containing a biologically derived material and a bioactive substance using an electrode pair to introduce the bioactive substance into the biologically derived material,
in which each of electrodes of the electrode pair has a boron-doped diamond membrane on a surface portion in contact with the suspension,
a flow channel for allowing the suspension to flow is provided, and
the electrode pair is installed with the flow channel interposed therebetween so that the boron-doped diamond membranes of the electrodes face each other.

Hereinafter, the electroporation device according to the first embodiment of the present invention will be described with reference to the accompanying drawings.

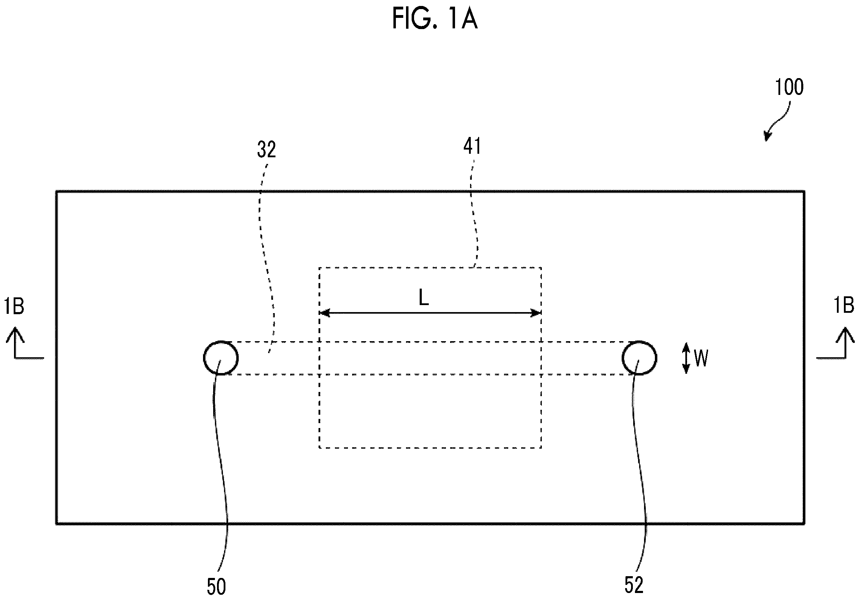

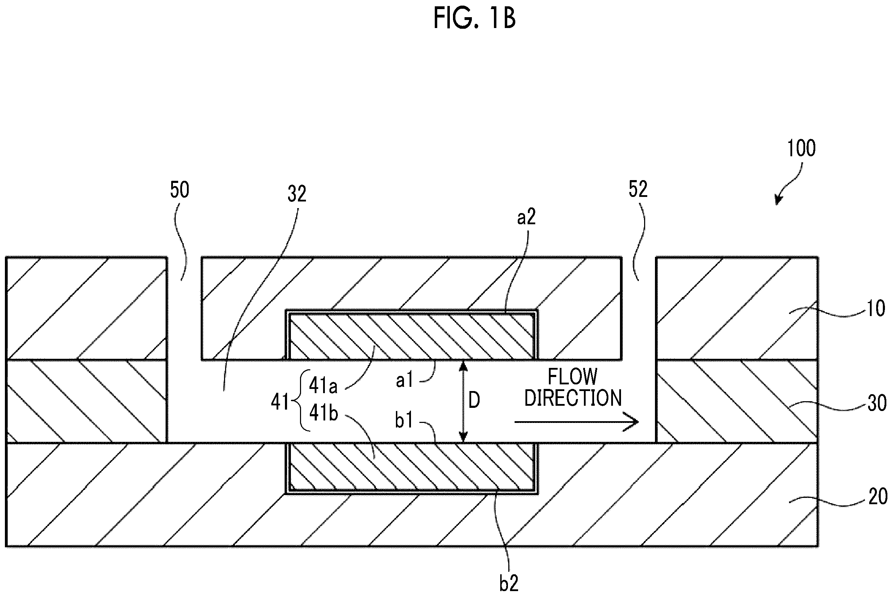

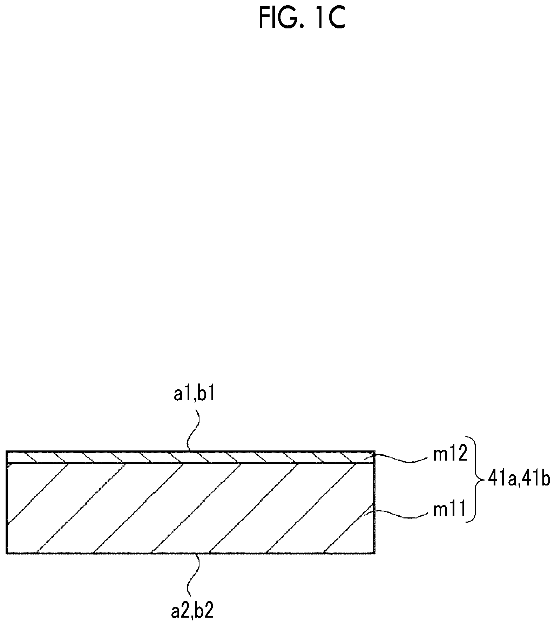

Fig. 1A is a top view showing an example of the electroporation device according to the first embodiment. Fig. 1B is a cross-sectional view taken along the line 1B-1B in Fig. 1A. Fig. 1C is a cross-sectional view of an upper electrode 41a and a lower electrode 41b.

As shown in Fig. 1B, an electroporation device 100 includes an upper electrode holding plate 10, a flow channel plate 30, and a lower electrode holding plate 20 in this order.

The flow channel plate 30 has an opening portion 32 serving as a flow channel. The upper electrode holding plate 10 has an inflow port 50 and an outflow port 52 that communicate with the opening portion 32 of the flow channel plate 30. As described above, since the flow channel plate 30 is interposed between the upper electrode holding plate 10 and the lower electrode holding plate 20, the flow channel 32 is formed by the opening portion 32 of the flow channel plate 30 and the inflow port 50 and the outflow port 52 of the upper electrode holding plate 10. In a case where the EP device is used, a suspension is injected from the inflow port 50, passes through the flow channel 32, and is discharged from the outflow port 52. A tube for a suspension may be connected to the inflow port 50 and the outflow port 52. Instead of the upper electrode holding plate 10, the lower electrode holding plate 20 may have the inflow port 50 and the outflow port 52, or the upper electrode holding plate 10 may have one of the inflow port 50 and the outflow port 52 and the lower electrode holding plate 20 may have the other of them. An O-ring for preventing the leakage of the suspension is preferably provided between the upper electrode holding plate 10 and the flow channel plate 30 and between the lower electrode holding plate 20 and the flow channel plate 30.

An upper electrode 41a is fitted in the upper electrode holding plate 10. In addition, a lower electrode 41b is fitted in the lower electrode holding plate 20. The upper electrode 41a and the lower electrode 41b constitute an electrode pair 41. A front surface portion a1 of the upper electrode and a front surface portion b1 of the lower electrode face each other via the flow channel 32. That is, the electrode pair 41 is installed with the flow channel 32 interposed therebetween. The front surface portions a1 and b1 are surface portions in contact with the flow channel 32, and are surface portions in contact with the suspension in a case where the EP device is used.

The method of fixing the upper electrode holding plate 10, the flow channel plate 30, and the lower electrode holding plate 20 is not particularly limited, and examples thereof include a method in which a through-hole for assembling is formed in each plate and the plates are fixed with screws and a method in which the plates are fixed with an adhesive.

The upper electrode holding plate 10 has a through-hole (not shown) for electrical connection to a rear surface portion a2, that is a surface portion on the side opposite to the front surface portion a1 of the upper electrode 41a. Similarly, the lower electrode holding plate 20 has a through-hole (not shown) for electrical connection to a rear surface portion b2, that is a surface portion on the side opposite to the front surface portion b1 of the lower electrode 41b. The upper electrode 41a and the lower electrode 41b are connected to an external power supply (not shown) through the through-holes.

As shown in Fig. 1C, each of the upper electrode 41a and the lower electrode 41b consists of a support m11 and a BDD membrane m12 that is a covering layer covering the support m11.

The upper electrode 41a and the lower electrode 41b have the BDD membrane m12 on the front surface portions a1 and b1 in contact with the flow channel 32, respectively. That is, each of the electrodes (the upper electrode 41a and the lower electrode 41b) of the electrode pair 41 has the BDD membrane m12 on the surface portion (liquid contact portion) in contact with the suspension.

### [Electrode Length L]

An electrode length L is not particularly limited, but is preferably 1 to 100 mm, and more preferably 10 to 50 mm due to the reason that the effects and the like of the present invention are more excellent.

### [Electrode Width W]

An electrode width W as the width of the upper electrode 41a and the width of the lower electrode 41b is not particularly limited, but is preferably 1 to 100 mm, more preferably 2 to 30 mm, and particularly preferably 5 to 25 mm due to the reason that the effects and the like of the present invention are more excellent.

From the viewpoint of excellent initial introduction efficiency, the electrode width W is preferably 5 to 25 mm.

The widths of the upper electrode 41a and the lower electrode 41b are not actual electrode widths. The width is the width of a portion of the electrode in contact with the flow channel, and corresponds to the width of the flow channel 32.

### [Inter-Electrode Distance D]

An inter-electrode distance D (the distance between the upper electrode 41a and the lower electrode 41b) of the electrode pair 41 is preferably 1 to 10 mm, and more preferably 2 to 5 mm due to the reason that the effects and the like of the present invention are more excellent.

The BDD membrane electrode generally has high efficiency in generating active oxygen species due to a high oxygen overvoltage and a wide potential window. Therefore, in a case where the inter-electrode distance D is excessively small, the ratio of an electrode surface area to an electrode space volume increases, the activation and the influence of the oxygen species on the suspension are increased, and the survival rate of a biologically derived material such as a cell may be decreased. In addition, from the viewpoint of the processing flow rate, the inter-electrode distance D is preferably 3 to 6 mm.

On the other hand, in a case where the inter-electrode distance D is excessively large, the electric field generation is excessive, heat generation or electric field concentration on an electrode end portion occurs, and thus boiling or discharge may occur, reducing the survival rate or the introduction efficiency. From the viewpoint of preventing heat generation, the inter-electrode distance D is preferably 1 to 3 mm.

The inter-electrode distance D corresponds to the thickness of the flow channel plate 30.

In the present specification, the inter-electrode distance D is also referred to as the inter-electrode gap or gap.

### [Electroporation]

Next, the electroporation using the electroporation device of the first embodiment will be described using the electroporation device 100.

In the electroporation device 100, a voltage (for example, a pulse voltage) is applied to the electrode pair 41 by an external power supply (not shown). Accordingly, an electric field is formed between the upper electrode 41a and the lower electrode 41b.

In a case where a suspension is injected from the inflow port 50, the injected suspension passes through the flow channel 32 and is discharged from the outflow port 52. Since an electric field is applied in a case where the suspension passes through the flow channel 32, fine holes are created in the membrane covering a surface of the biologically derived material in the suspension, whereby the permeability of the membrane increases. As a result, the bioactive substance in the suspension is introduced into the biologically derived material by diffusion or electrophoresis.

### <Suspension>

The suspension used for electroporation contains a biologically derived material and a bioactive substance.

### (Biologically Derived Material)

The biologically derived material is not particularly limited, and specific examples thereof include a cell, a cell organelle, an intracellular granule, and a vesicle. Among these, the biologically derived material is preferably a cell, more preferably an animal cell, still more preferably a mammalian cell, and most preferably a human-derived cell due to the reason that the effects and the like of the present invention are more excellent. Specific examples of the cell include human T-cell, human embryonic kidney (HEK) 293 cell, A549, SF9, EB66, Daudi, Hela, Vero, MDCK, baby hamster kidney (BHK), Chinese hamster ovary (CHO), NS0, SP2/0, and hybridoma. In view of medicinal chemical manufacturing, gene introduction into HEK 293 cells or CHO cells is most commonly used.

### (Bioactive Substance)

The bioactive substance is a substance such as DNA, RNA, and protein, that exerts some action on a biologically derived material by being introduced into the biologically derived material. The bioactive substance is, for example, plasmid, linear DNA, mRNA, or protein, preferably plasmid, mRNA, or linear DNA, and particularly preferably plasmid.

### <Voltage>

In general, the voltage is set according to the inter-electrode gap so that an electric field applied to the suspension between the electrodes reaches a desired electric field strength.

In a case where electric resistances of the electrode in a thickness direction and an in-plane direction are sufficiently small, the value of the strength of the electric field applied to the suspension between the electrodes is obtained by dividing the voltage applied from an external power supply by the thickness of the inter-electrode gap, and a uniform electric field is formed regardless of the position of the liquid contact surface.

On the other hand, in a case where electric resistance values of the electrode in the thickness direction and the in-plane direction are high, a part of the voltage applied from the external power supply is consumed in the electrode portion, and thus a decrease in effective strength of the electric field applied to the suspension or a distribution of the electric field strength in the liquid contact surface occurs, which causes a decrease in electroporation efficiency.

The optimum value of the electric field varies depending on the kind of the biologically derived material and the like, but is usually about 1,000 to 2,000 V/cm.

The voltage is preferably a pulse voltage. In addition, a bipolar pulse (alternating between a positive pulse and a negative pulse) may be provided in order to make the electrode reaction (gas generation by electrolysis, electrode degradation, and the like) uniform.

### <Pulse Width>

The optimum value of a pulse width varies depending on the kind of the biologically derived material and the like, but is usually 0.1 to 100 ms (milliseconds), and preferably about 1 to 10 ms.

### <Pulse Period (Pulse Interval)>

The pulse period is preferably synchronized with (an integral multiple of) a time (determined by the flow rate and the flow channel cross-sectional area (electrode width W × inter-electrode distance D)) in which the biologically derived material passes through the electrode (electrode length L). For example, a pulse voltage is applied an average of 1 to 5 times, and preferably once while the biologically derived material passes through the electrode (electrode length L).

### [Modification Example]

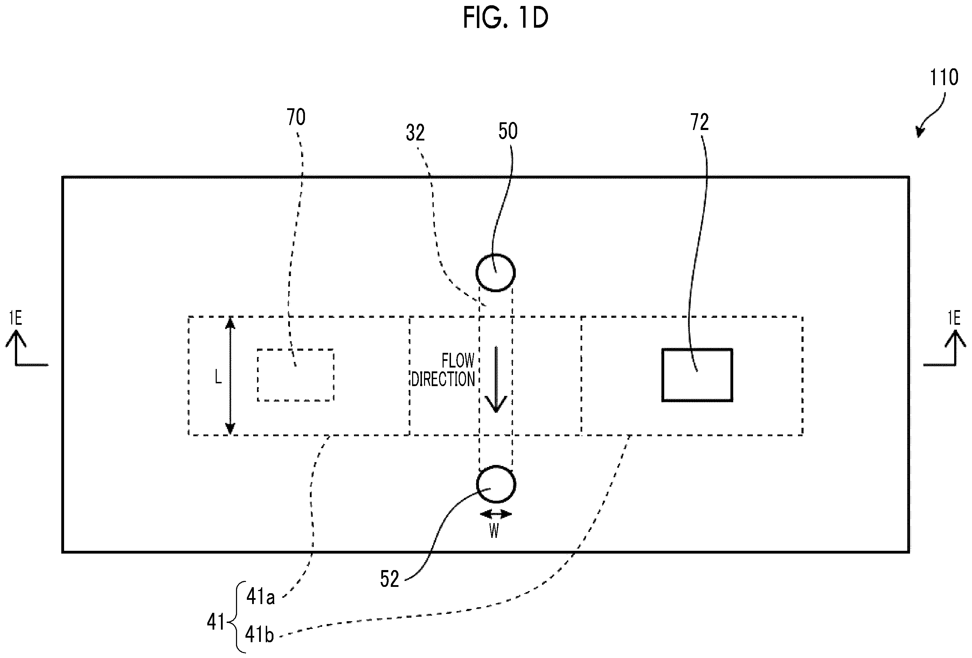

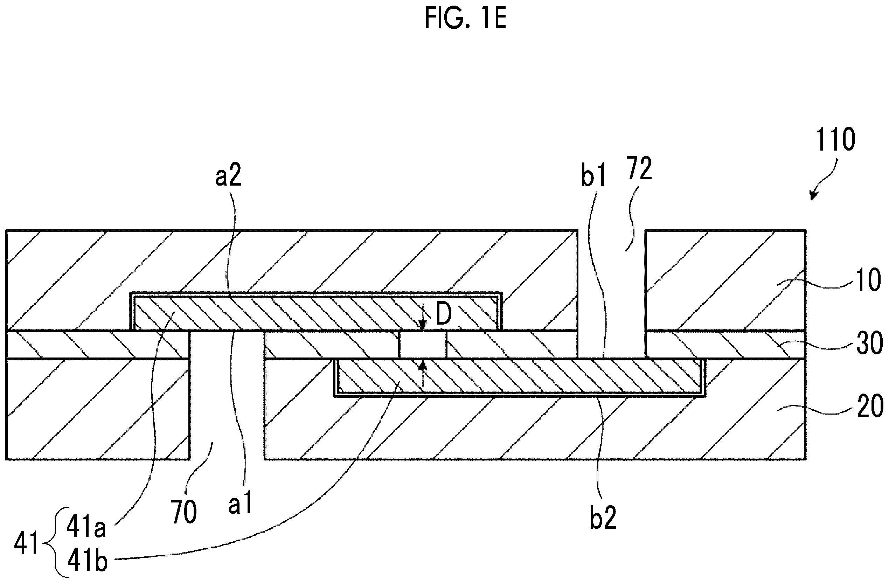

Fig. 1D is a top view showing a modification example of the electroporation device according to the first embodiment. Fig. 1E is a cross-sectional view taken along the line 1E-1E in Fig. 1D.

As described above, in the electroporation device 100 shown in Figs. 1A and 1B, an external power supply is connected to the rear surface portion a2 of the upper electrode 41a and the rear surface portion b2 of the lower electrode 41b through through-holes. However, in an electroporation device 110 shown in Figs. 1D and 1E, an external power supply (not shown) is connected to a front surface portion a1 of an upper electrode 41a through a through-hole 70, and similarly, the external power supply (not shown) is connected to a front surface portion b1 of a lower electrode 41b through a through-hole 72.

Here, as described above, the upper electrode 41a and the lower electrode 41b have the BDD membrane m12 on the front surface portions a1 and b1, respectively. That is, in the electroporation device 110, the external power supply is directly connected to the BDD membranes m12 of the upper electrode 41a and the lower electrode 41b.

The electroporation device 110 is useful, for example, in a case where the support m11 is a material (for example, Si) other than a metal.

### [Second Embodiment]

Next, an electroporation device according to a second embodiment of the present invention will be described.

The second embodiment is the same as the above-described first embodiment, except that the electrode pair is changed.

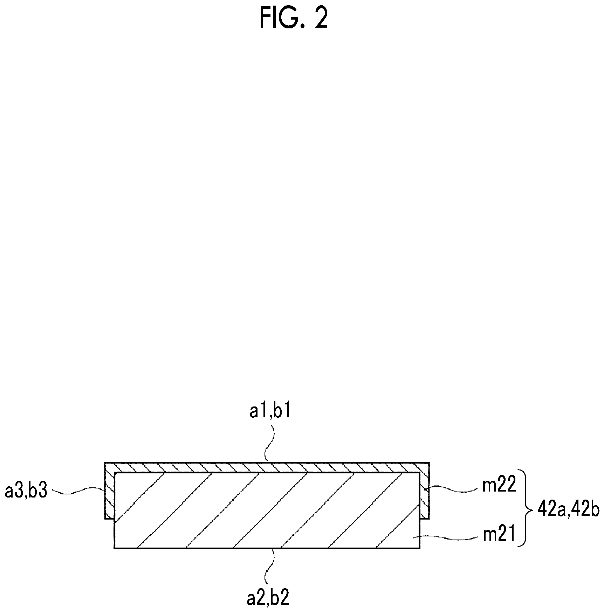

Fig. 2 is a cross-sectional view of an upper electrode 42a and a lower electrode 42b in an example of electroporation according to the second embodiment.

As shown in Fig. 2, each of the upper electrode 42a and the lower electrode 42b consists of a support m21 and a BDD membrane m22 that is a covering layer covering the support m21. The upper electrode 42a and the lower electrode 42b constitute an electrode pair 42.

The upper electrode 42a and the lower electrode 42b have the BDD membrane m22 on front surface portions a1 and b1 in contact with a flow channel 32, respectively. That is, each of the electrodes (the upper electrode 42a and the lower electrode 42b) of the electrode pair 42 has the BDD membrane m22 on the surface portion (liquid contact portion) in contact with a suspension.

Further, the upper electrode 42a and the lower electrode 42b have the BDD membrane m22 not only on the front surface portions a1 and b1 but also on portions that are equal to or more than half or 0.5 mm or more (preferably 2 mm or more) of side surface portions a3 and b3 of the electrode in a thickness direction (corresponding to a direction from one electrode to the other electrode of the electrode pair 42) continuously from the front surface portions a1 and b1, respectively. 60% or more of the area of the side surface portion is preferably covered, more preferably 80% or more, and still more preferably 95% or more.

In a case where the EP device is used, an electric field or heat is likely to be concentrated on a corner portion of the BDD membrane electrode or a side surface portion. In the second embodiment, since a site where such an electric field or heat is likely to be concentrated has the BDD membrane, the introduction efficiency is less likely to decrease than in the above-described first embodiment.

### [Third Embodiment]

Next, an electroporation device according to a third embodiment of the present invention will be described.

The third embodiment is the same as the above-described first embodiment, except that the electrode pair is changed.

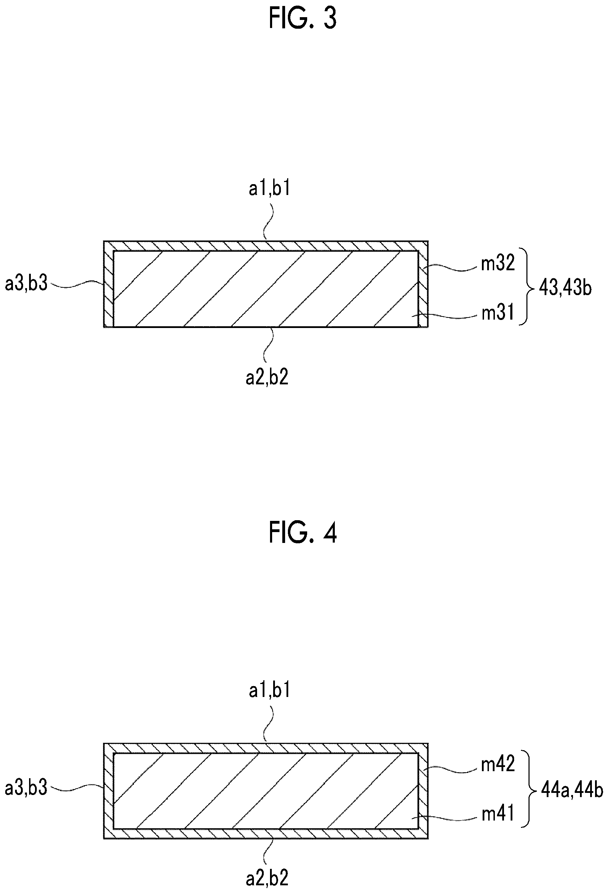

Fig. 3 is a cross-sectional view of an upper electrode 43a and a lower electrode 43b in an example of electroporation according to the third embodiment.

As shown in Fig. 3, each of the upper electrode 43a and the lower electrode 43b consists of a support m31 and a BDD membrane m32 that is a covering layer covering the support m31. The upper electrode 43a and the lower electrode 43b constitute an electrode pair 43.

The upper electrode 43a and the lower electrode 43b have the BDD membrane m32 on front surface portions a1 and b1 in contact with a flow channel 32, respectively. That is, each of the electrodes (the upper electrode 43a and the lower electrode 43b) of the electrode pair 43 has the BDD membrane m32 on the surface portion (liquid contact portion) in contact with a suspension.

Further, the upper electrode 43a and the lower electrode 43b have the BDD membrane m32 not only on the front surface portions a1 and b1 but also on side surface portions a3 and b3 continuously from the front surface portions a1 and b1, respectively. Therefore, the introduction efficiency is less likely to decrease than in the above-described first embodiment.

### [Fourth Embodiment]

Next, an electroporation device according to a fourth embodiment of the present invention will be described.

The fourth embodiment is the same as the above-described first embodiment, except that the electrode pair is changed.

Fig. 4 is a cross-sectional view of an upper electrode 44a and a lower electrode 44b in an example of electroporation according to the fourth embodiment.

As shown in Fig. 4, each of the upper electrode 44a and the lower electrode 44b consists of a support m41 and a BDD membrane m42 that is a covering layer covering the support m41. The upper electrode 44a and the lower electrode 44b constitute an electrode pair 44.

The upper electrode 44a and the lower electrode 44b have the BDD membrane m42 on front surface portions a1 and b1 in contact with a flow channel 32, respectively. That is, each of the electrodes (the upper electrode 44a and the lower electrode 44b) of the electrode pair 44 has the BDD membrane m42 on the surface portion (liquid contact portion) in contact with a suspension.

Further, the upper electrode 44a and the lower electrode 44b have the BDD membrane m42 not only on the front surface portions a1 and b1 but also on side surface portions a3 and b3 and rear surface portions a2 and b2 continuously from the front surface portions a1 and b1, respectively. Therefore, the introduction efficiency is less likely to decrease than in the above-described first embodiment.

### [Fifth Embodiment]

Next, an electroporation device according to a fifth embodiment of the present invention will be described.

The fifth embodiment is the same as the above-described first to fourth embodiments, except a cooling mechanism that cools an electrode pair is provided. Examples of the cooling mechanism include a refrigerant circulation device and a heat exchanger.

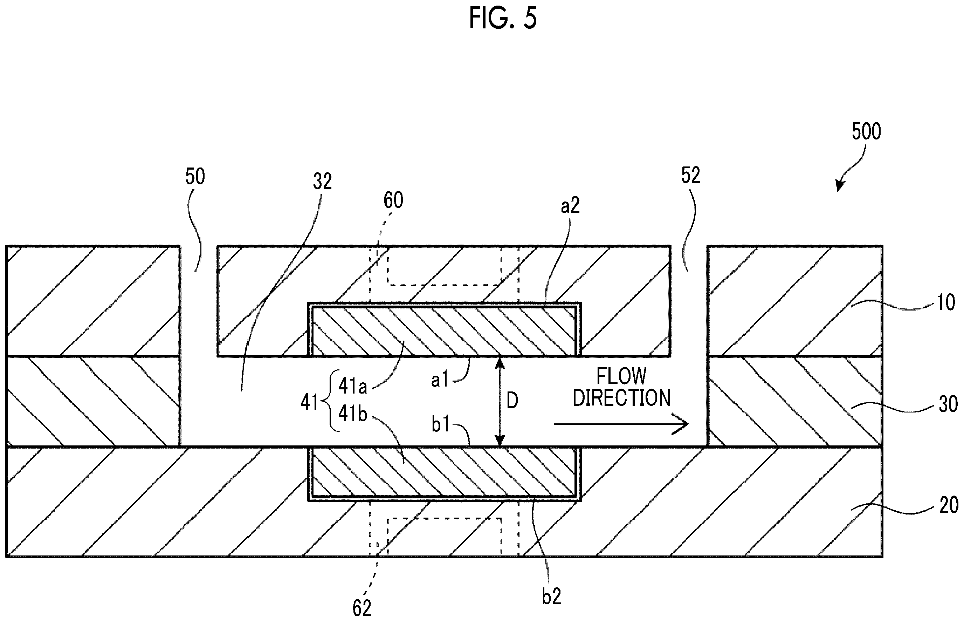

Fig. 5 is a cross-sectional view showing an example of the electroporation device according to the fifth embodiment.

An electroporation device 500 shown in Fig. 5 is the same as the electroporation device 100 shown in Figs. 1A and 1B, except that a flow channel 60 for upper electrode cooling and a flow channel 62 for lower electrode cooling are added.

As shown in Fig. 5, the flow channel 60 for upper electrode cooling is formed on the rear surface portion a2 side of an upper electrode 41a in the electroporation device 500. Similarly, the flow channel 62 for lower electrode cooling is formed on the rear surface portion b2 side of a lower electrode 41b. A tube for a refrigerant may be attached to each of inlets and outlets of the flow channel 60 for upper electrode cooling and the flow channel 62 for lower electrode cooling.

In a case where the EP device is used, the electrode pair generates heat. As a result, the biologically derived material and the bioactive substance in a suspension may be damaged, and this may lead to a decrease in survival rate and introduction efficiency. In the fifth embodiment, since it is possible to suppress the heat generation of the electrode pair by allowing a refrigerant (cooling water or the like) to flow through the flow channel 60 for upper electrode cooling and the flow channel 62 for lower electrode cooling, it is possible to further suppress a decrease in survival rate and introduction efficiency.

### [Sixth Embodiment]

An electroporation device according to a sixth embodiment of the present invention is
an electroporation device that is used for batch electroporation in which an electric field is applied to a suspension containing a biologically derived material and a bioactive substance using an electrode pair to introduce the bioactive substance into the biologically derived material,
in which each of electrodes of the electrode pair has a boron-doped diamond membrane on a surface portion in contact with the suspension,
a container for accommodating the suspension is provided, and
the electrode pair is installed on an inner surface of the container so that the boron-doped diamond membranes of the electrodes face each other.

The electroporation device according to the sixth embodiment may be a single batch type or a repeated batch type, but is preferably a repeated batch type.

### [Members]

Next, members used in the electroporation device according to the embodiment of the present invention will be described.

### [BDD Membrane Electrode]

As described above, in the present invention, an electrode (BDD membrane electrode) having a boron-doped diamond membrane (BDD membrane) on a liquid contact portion is used.

Due to the reason that the effects and the like of the present invention are more excellent, the BDD membrane electrode preferably includes a support and the BDD membrane covering the support.

### <BDD Membrane>

The BDD membrane is a diamond membrane doped with boron and has conductivity.

### (B Content)

Due to the reason that the effects and the like of the present invention are more excellent, the boron content (hereinafter, also referred to as a "B content") of the BDD membrane is preferably 100 to 10,000 ppm, more preferably 1,000 to 40,000 ppm, and still more preferably 2,000 to 10,000 ppm.

The B content is preferably 100 ppm or more from the viewpoint that the resistivity of the BDD membrane is decreased, and as a result, the electric field applied to the suspension increases, the ohmic loss (heat generation) in the electrode decreases, and a voltage distribution in the electrode surface is suppressed. In addition, from the viewpoint of membrane hardness and durability, the B content is preferably 10,000 ppm or less.

The boron content of the BDD membrane can be calculated from a ratio of elements C and B measured by cutting a substrate on which a membrane has been formed in the same membrane formation batch into a small piece and by performing X-ray photoelectron spectroscopy (XPS).

### (Membrane Thickness)

From the viewpoint that the effects of the present invention are more excellent, the membrane thickness of the BDD membrane is preferably 2 to 20 µm, and more preferably 5 to 10 µm .

The membrane thickness of the BDD membrane is preferably 2 µm or more from the viewpoint of durability due to pinholes or the like. In addition, the membrane thickness of the BDD membrane is preferably 20 µm or less from the viewpoint of suppressing the membrane stress and improving the adhesiveness of the BDD membrane.

The membrane thickness of the BDD membrane can be calculated assuming that the specific gravity of diamond is 3.52 g/cm³ from the difference in mass before and after membrane formation.

### (Resistivity)

Due to the reason that the effects and the like of the present invention are more excellent, the resistivity of the BDD membrane is preferably 1 × 10⁻³ to 1 × 10⁻¹ Ω·cm, and more preferably 2 × 10⁻³ to 1 × 10⁻² Ω·cm.

The resistivity is preferably 1 × 10⁻¹ Ω·cm or less from the viewpoint that the electric field applied to the suspension increases, the ohmic loss (heat generation) in the electrode decreases, and a voltage distribution in the electrode surface is suppressed. In addition, from the viewpoint of membrane hardness and durability, the resistivity is preferably 1 × 10⁻³ Ω·cm or more.

The resistivity of the BDD membrane can be calculated by dividing, by a membrane thickness, a measured sheet resistance value of a BDD membrane on a high-resistance silicon substrate on which the membrane has been formed in the same membrane formation batch.

### <Support>

The support is not particularly limited, and may be a substrate (for example, a silicon (Si) substrate or a SiC substrate) other than a metal or a metal substrate. However, due to the reason that the effects and the like of the present invention are more excellent, a metal substrate is preferable.

### (Metal Material)

The metal material of the metal substrate is not particularly limited, but from the viewpoint of membrane quality, adhesiveness to the BDD membrane, and thermal conductivity, and due to the reason that the effects and the like of the present invention are more excellent, molybdenum (Mo), niobium (Nb), tungsten (W), or titanium (Ti) is preferable, and Mo is more preferable.

### (Thickness)

The thickness of the support is not particularly limited, but is preferably 0.5 to 10 mm, and more preferably 1 to 5 mm due to the reason that the effects and the like of the present invention are more excellent.

### <Thickness>

The thickness of the BDD membrane electrode is not particularly limited, but is preferably 0.5 to 10 mm, and more preferably 1 to 5 mm due to the reason that the effects and the like of the present invention are more excellent.

### <Manufacturing Method>

The method of forming the BDD membrane on the support is not particularly limited, and a known diamond membrane forming method can be used. Examples thereof include a hot filament chemical vapor deposition (CVD) method, a microwave plasma CVD method, an ion beam irradiation method, and a plating method. Among these, a hot filament CVD method is preferable due to the reason that the effects and the like of the present invention are more excellent in an electroporation device to be obtained.

Specific examples of the method of manufacturing the BDD membrane using the hot filament CVD method include a method of forming a BDD membrane on a support by supplying a mixed gas obtained by mixing a carbon source and a boron source with high-purity hydrogen using a hot filament CVD device.

Examples of the carbon source include methane and ethane. In addition, examples of the boron source include trimethyl borate (B(OCH₃)₃), triethyl borate (B(OC₂H₅)₃), and diborane.

### [Electrode Holding Member]

The materials of the upper electrode holding member and the lower electrode holding member are not particularly limited, but a material having high workability and capable of withstanding autoclave sterilization at 121°C for 20 minutes or longer is preferable. Examples of such a material include polycarbonate and polypropylene.

### [Flow Channel Plate]

The material of the flow channel plate is not particularly limited, but a fluororesin material such as TEFLON (registered trademark) is preferable from the viewpoint of reducing the adhesion of a biologically derived material such as a cell and preventing damage due to heat generation or discharge during electroporation.

In a case where it is difficult to prevent liquid leakage by an O-ring as in the above-described modification example, a material (for example, polydimethylsiloxane (PDMS)) having flexibility and slight pressure-sensitive adhesiveness can be used for the flow channel plate and can also serve as a sealing member.

### Examples

Hereinafter, the present invention will be described in more detail with reference to examples; however, the present invention is not limited thereto.

### [Manufacturing of Electroporation Device]

Electroporation devices were manufactured as follows.

### [Example 3]

An EP device as shown in Fig. 5 was manufactured.

### <Production of Upper Electrode Holding Plate and Lower Electrode Holding Plate>

An upper electrode holding plate and a lower electrode holding plate were manufactured using polycarbonate.

A groove for fitting an upper electrode and a through-hole for electrical connection to the upper electrode were formed in the upper electrode holding plate. Similarly, a groove for fitting a lower electrode and a through-hole for electrical connection to the lower electrode were formed in the lower electrode holding plate.

In addition, through-holes for an inflow port and an outflow port were formed in the upper electrode holding plate. A flow channel tube was attached to the inflow port and the outflow port.

In addition, a flow channel for electrode cooling was formed in both of the upper electrode holding plate and the lower electrode holding plate. A tube for cooling water was attached to the flow channel for electrode cooling.

In addition, a through-hole for assembling and a groove for an O-ring for preventing liquid leakage were formed in both of the upper electrode holding plate and the lower electrode holding plate.

### <Production of Flow Channel Plate>

A flow channel plate was manufactured using TEFLON (registered trademark).

In the flow channel plate, an opening portion (width: 20 mm) serving as a flow channel and a through-hole for assembling were formed. The flow channel plate has a thickness of 2 mm.

### <Production of Electrode>

A BDD membrane was formed from one surface to side surfaces of a Mo substrate (support) (length in flow direction: 20 mm, thickness: 2 mm) by a hot filament chemical vapor deposition (CVD) method using a mixed gas of hydrogen, methane, and trimethyl borate (membrane forming temperature: 1,000°C). By performing external shape working thereon, a Mo substrate (BDD membrane electrode) was obtained in which only one surface of the support was covered with the BDD membrane as shown in Fig. 1C. The obtained BDD membrane electrode consisted of a support and a BDD membrane covering the support, and had the BDD membrane as a covering layer only on one surface portion as shown in Fig. 1C (electrode length L: 20 mm). The BDD membrane had a membrane thickness of 2 µm.

### <Device Assembling>

The upper electrode was fitted into the groove of the upper electrode holding plate so that the BDD membrane was exposed. In addition, an O-ring was fitted into the groove. Similarly, the lower electrode was fitted into the groove of the lower electrode holding plate so that the BDD membrane was exposed, and an O-ring was fitted into the groove. The flow channel plate was sandwiched between the upper electrode holding plate and the lower electrode holding plate and fixed with screws so that the BDD membrane of the upper electrode and the BDD membrane of the lower electrode faced each other.

In this manner, an EP device was produced. In the obtained EP device, the electrode pair is installed with the flow channel interposed therebetween so that the BDD membranes of the electrodes face each other.

### [Example 4]

An EP device was produced according to the same procedure as in Example 3, except that in the production of the electrode, the membrane forming condition (membrane forming time) was changed so that the membrane thickness of the BDD membrane was 10 µm.

### [Example 5]

An EP device was produced according to the same procedure as in Example 4, except that in the production of the electrode, the substrate was subjected to the external shape working before the BDD membrane was formed. In Example 5, the upper electrode and the lower electrode are Mo substrates (BDD membrane electrodes) covered with a BDD membrane from one surface to side surfaces as shown in Fig. 3. The obtained BDD membrane electrode consists of a support and a BDD membrane covering the support, and has the BDD membrane as a covering layer not only on one surface portion but also on side surface portions continuously from the surface portion as shown in Fig. 3.

### [Example 6]

An EP device was produced according to the same procedure as in Example 5, except that the thickness of the flow channel plate was changed to 4 mm.

### [Example 1]

An EP device as shown in Figs. 1D and 1E was manufactured.

Polycarbonate was used for an upper electrode holding plate and a lower electrode holding plate as in Example 3.

Meanwhile, since it was difficult to prevent liquid leakage with an O-ring, polydimethylsiloxane (PDMS) having flexibility and slight pressure-sensitive adhesiveness was used for a flow channel plate (width of opening portion serving as flow channel: 2 mm). The PDMS is used as a flow channel plate and a sealing member.

A BDD membrane electrode was produced according to the same procedure as in Example 3, except that a Si substrate (thickness: 0.7 mm) was used as a support instead of the Mo substrate.

### [Example 2]

An EP device was produced according to the same procedure as in Example 1, except that the width of the opening portion serving as the flow channel of the flow channel plate was changed to 20 mm.

### [Comparative Example 1]

An EP device was produced according to the same procedure as in Example 3, except that the width of the opening portion serving as the flow channel was changed to 2 mm and the following Pt membrane electrode was used as the upper electrode and the lower electrode. In the obtained EP device, the electrode pair is installed with the flow channel interposed therebetween so that the Pt membranes of the electrodes face each other.

### <Pt Membrane Electrode>

A Cu substrate (support) (thickness: 2 mm) was worked into an electrode shape, deburred, and surface-cleaned. A Cu plating layer having a thickness of 10 µm was formed as an underlayer smoothing layer, an Au plating layer having a thickness of 0.1 µm was formed as an adhesiveness improving layer, and a Pt plating layer (Pt membrane) having a thickness of 2 µm was formed as a surface layer.

### [Comparative Example 2]

An EP device was produced according to the same procedure as in Comparative Example 1, except that the width of the opening portion serving as the flow channel was changed to 20 mm.

### [Comparative Examples 3 and 4]

An EP device was produced according to the same procedure as in Comparative Example 1, except that the thickness of the flow channel plate was changed to 4 mm.

### [Comparative Example 5]

An EP device was produced according to the same procedure as in Example 3, except that a Cu substrate (electrode length L: 20 mm, thickness: 2 mm, no covering layer) was used as the upper electrode and the lower electrode.

### [Comparative Example 6]

An EP device was produced according to the same procedure as in Example 3, except that the following diamond membrane electrode was used as the upper electrode and the lower electrode. In the obtained EP device, the electrode pair is installed with the flow channel interposed therebetween so that the diamond membranes of the electrodes face each other.

### <Diamond Membrane Electrode>

A diamond membrane was formed from one surface to side surfaces of a Mo substrate (support) (length in flow direction: 20 mm, thickness: 2 mm) by a hot filament chemical vapor deposition (CVD) method using a mixed gas of hydrogen and methane (membrane forming temperature: 1,000°C). The obtained diamond membrane electrode consists of a support and a diamond membrane covering the support, and has the diamond membrane as a covering layer not only on one surface portion but also on side surface portions continuously from the surface portion.

### [Comparative Example 7]

An EP device was produced according to the same procedure as in Example 3, except that the following conductive polymer electrode was used as the upper electrode and the lower electrode.

### <Conductive Polymer Electrode>

With reference to JP2005-526499A, 80% of polycarbonate, 20% of carbon black, and an additive were mixed and the mixture was formed into an electrode shape to obtain a conductive polymer electrode (electrode length L: 20 mm, thickness: 2 mm).

### [Comparative Example 8]

An EP device was produced according to the same procedure as in Example 6, except that the conductive polymer electrode was used as the upper electrode and the lower electrode.

Regarding the obtained EP devices, the following items are shown in Table 1.
· Covering Layer: covering layer of electrode (kind, covering site, B content, resistivity, and membrane thickness)
· Support: support of electrode (material, thickness)
· Electrode: electrode length L, electrode width W, and gap (inter-electrode distance D) of each electrode (upper electrode and lower electrode)

As described above, the widths of the upper electrode and the lower electrode are not actual electrode widths. The width is the width of a portion of the electrode in contact with the flow channel, and corresponds to the width of the flow channel.

### [Electroporation]

Electroporation was performed using the obtained electroporation device.

### [Preparation of Suspension]

A cell suspension (concentration: 40 M (× 10⁶) cells/mL) of proliferated and cultured HEK293 cells (corresponding to a biologically derived material) was prepared.

pmaxGFP plasmid (corresponding to a bioactive substance) manufactured by Lonza was added to the obtained cell suspension so that a concentration of 30 µg/mL was reached. In this way, a suspension containing the HEK293 cells and the GFP plasmid was obtained.

### [Electroporation]

The obtained EP device was packed in a bag for an autoclave, and autoclave sterilization (121°C for 20 to 60 minutes) was carried out.

In the safety cabinet (sterile environment), the inflow port of the sterilized EP device was connected to a suspension supply system, and the outflow port was connected to a recovery system. In addition, the electrode pair of the EP device was connected to a pulse power supply.

The suspension prepared as described above was fed from the inflow port at a flow rate shown in Table 1 to perform electroporation. The reason why the flow rate is changed is to keep the average flow rate constant. Specifically, the flow rate was changed in accordance with the cross-sectional area of the flow channel.

In addition, the voltage and the pulse width for the electroporation were adjusted so that an electric field of 1,375 V/cm was produced. In addition, the pulse period (pulse interval) was synchronized with a central flow velocity so that the electric field was applied to the suspension once. This is specifically shown in Table 2 below. In order to make the electrode reaction uniform, a bipolar pulse (alternating between a positive pulse and a negative pulse) was used.

In addition, in Example 6 and Comparative Examples 4, 7, and 8, pure water (4°C) was fed (100 mL/min) to the flow channel for electrode cooling in order to cool the electrode.

### [Evaluation]

The suspension after electroporation during 1st to 25th pulses (25 pulses) (15 seconds) was collected, and the suspension after subsequent electroporation during 26th to 100th pulses (75 pulses) was discarded. The same operation was repeated after a 101st pulse.

The recovered suspension was diluted to 2 M (× 10⁶) cells/mL, and then 2 mL thereof were seeded in a well plate, accommodated in an incubator having a CO₂ concentration of 8% and an atmospheric temperature of 37°C, and statically cultured for 24 hours.

A cell concentration X after culturing for 24 hours was acquired using Vi-CELL XR (Beckman Coulter, Inc.), and a proportion Y of the gene-introduced cells was acquired using BD FACS Calibur (Becton, Dickinson and Company).

Then, as a proportion of the gene-introduced cells obtained after culturing for 24 hours to the cells subjected to electroporation, an introduction efficiency (= cell concentration X × cell proportion Y ÷ 2 M cells/mL × 100) (%) was calculated.

### [Initial Introduction Efficiency]

The introduction efficiency during 1st to 25th pulses (25 pulses) is shown in Table 1.

The higher the initial introduction efficiency is, the more preferable it is, and the initial introduction efficiency is preferably 20% or more in practice.

### [Endurance Pulse Number]

The maximum cumulative number of pulses (endurance pulse number) at which the introduction efficiency was maintained to 90% or more of the initial introduction efficiency was obtained. For example, in a case where a maintenance rate (introduction efficiency/initial introduction efficiency) was 90% or more up to 801st to 825th pulses (25 pulses), but a maintenance rate during 901st to 925th pulses (25 pulses) was less than 90%, the endurance pulse number was 800. In a case where the initial introduction efficiency was less than 20%, the endurance pulse number was not obtained (Comparative Examples 3, 5, 6, and 8).

The results are shown in Table 1. The larger the endurance pulse number, the less likely the introduction efficiency is to decrease. The endurance pulse number is preferably 1,000 or more in practice.

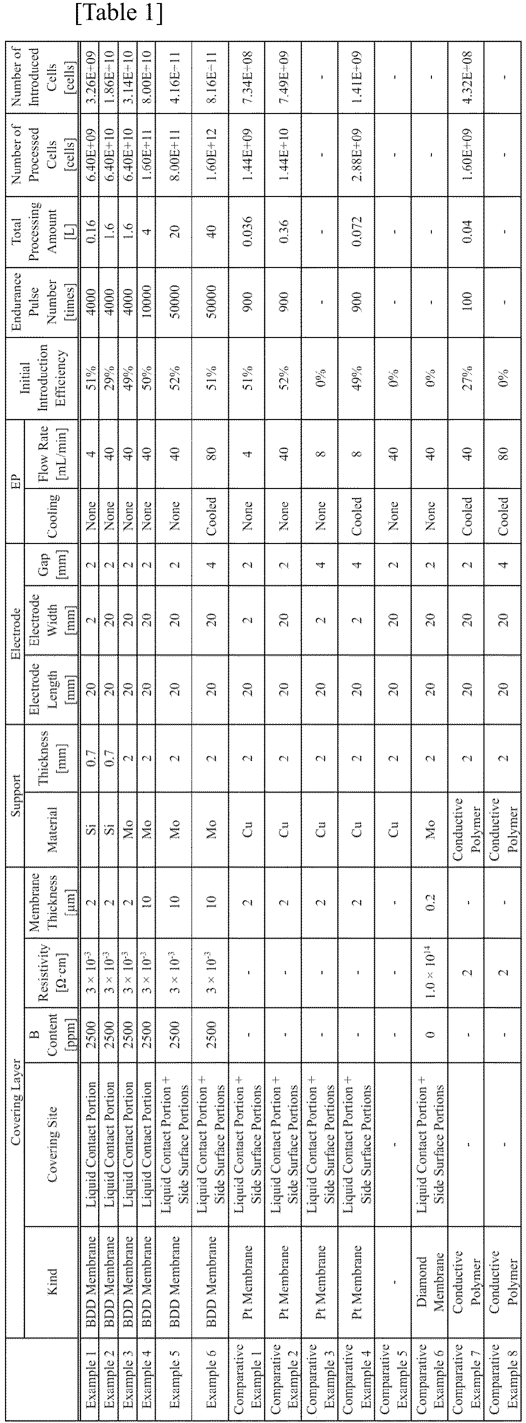

**[Table 2]**

| Electrode Structure | | | EP Conditions | | | | | For Evaluation | | | Processing Flow Rate Per Pulse [mL/times] |
|---|---|---|---|---|---|---|---|---|---|---|---|
| Electrode Length [mm] | Electrode Width [mm] | Gap [mm] | Flow Rate [mL/min] | Electric Field [V/cm] | Voltage [V] | Pulse Width [ms] | Pulse Period [ms] | Processing Amount [mL] | Processing Time [see] | Pulse Number [times] | |
| 20 | 2 | 2 | 4 | 1375 | 275 | 5 | 600 | 1 | 15 | 25 | 0.04 |
| 20 | 20 | 2 | 40 | 1375 | 275 | 5 | 600 | 10 | 15 | 25 | 0.4 |
| 20 | 2 | 4 | 8 | 1375 | 550 | 5 | 600 | 2 | 15 | 25 | 0.08 |
| 20 | 20 | 4 | 80 | 1375 | 550 | 5 | 600 | 20 | 15 | 25 | 0.8 |

In Table 1, the number of introduced cells is the result of multiplying the initial introduction efficiency by the number of processed cells. In addition, E represents a power of 10 in Table 1.

In all of Examples 1 to 6 in which the electrode was a BDD membrane electrode, high initial introduction efficiency and a high endurance pulse number were exhibited.

From the comparison between Example 1 and Comparative Example 1 (electrode width: 2 mm, gap: 2 mm), the endurance pulse number was higher in Example 1 in which the electrode was a BDD membrane electrode than in Comparative Example 1 in which the electrode was a Pt membrane electrode. Similarly, from the comparison between Examples 2 and 3 and Comparative Example 2 (electrode width: 20 mm, gap: 2 mm), the endurance pulse number was higher in Examples 2 and 3 in which the electrode was a BDD membrane electrode than in Comparative Example 2 in which the electrode was a Pt membrane electrode. In Comparative Example 5 in which the electrode was a copper substrate and Comparative Example 6 in which the electrode was a diamond membrane electrode, the initial introduction efficiency was insufficient. In addition, in Comparative Examples 7 and 8 in which the electrode was a conductive polymer, the endurance pulse number or the initial introduction efficiency was insufficient.

From the comparison between Example 2 and Example 3 (the comparison between the aspects in which the supports were different), the initial introduction efficiency was higher in Example 3 in which the support of the electrode was a Mo substrate than in Example 2 in which the support of the electrode was a silicon substrate (by changing the silicon substrate having high electric resistance to the metal Mo substrate, the electric resistance in the in-plane direction of the electrode was reduced, the electric field strength in the liquid contact surface was uniformized even in a case where the liquid contact area of the electrode was large, whereby the introduction efficiency was improved).

In addition, from the comparison between Example 3 and Example 4 (the comparison between the aspects in which the BDD membranes had different membrane thicknesses), the endurance pulse number was higher in Example 4 in which the membrane thickness of the BDD membrane was 5 µm or more (The size and density of pinholes of the BDD membrane generated due to the surface unevenness or particle defects of the base substrate decrease as the membrane thickness is increased. As a result, it is presumed that even in a case where the damage to the BDD membrane progresses with an increase in cumulative number of pulses applied, the covering property of the BDD membrane with respect to the base metal is sufficiently maintained, and the elution of the metal derived from the base substrate is suppressed).

In addition, from the comparison between Examples 4, 5, and 6 (the comparison between the aspects in which the covering sites were different), the endurance pulse number was higher in Example 5 and Example 6 in which the BDD membrane electrode had the BDD membrane not only on the liquid contact portion but also on the side surface portions continuously from the liquid contact portion (In Example 4, the BDD membrane at the electrode corner portion disappeared after application of 10,000 pulses, and the exposure of the base substrate was observed. The exposure of the base at the corner portion was expanded by further continuous application of pulses. On the other hand, in Example 5 (and also in Example 6), the base substrate at the electrode corner portion was not exposed even in a case where 50,000 pulses were applied, and the gene introduction efficiency did not decrease. In Example 6, the gap was larger than in Example 5 (the number of processed cells was larger) and more severe conditions were made. However, the endurance pulse number was maintained by cooling the electrodes.

In addition, in a case where electroporation was carried out using CHO cells instead of HEK293 cells as a biologically derived material and evaluation was performed in the same manner as in the above-described examples and comparative examples, the same tendency as in the above-described examples and comparative examples was found.

### Explanation of References

10: upper electrode holding plate
20: lower electrode holding plate
30: flow channel plate
32: opening portion (flow channel)
41: electrode pair
41a: upper electrode
41b: lower electrode
42: electrode pair
42a: upper electrode
42b: lower electrode
43: electrode pair
43a: upper electrode
43b: lower electrode
44: electrode pair
44a: upper electrode
44b: lower electrode
50: inflow port
52: outflow port
60: flow channel for upper electrode cooling
62: flow channel for lower electrode cooling
70: through-hole
72: through-hole
100: electroporation device
110: electroporation device
500: electroporation device
a1: front surface portion of upper electrode
a2: rear surface portion of upper electrode
a3: side surface portion of upper electrode
b1: front surface portion of lower electrode
b2: rear surface portion of lower electrode
b3: side surface portion of lower electrode
m11: support
m12: BDD membrane
m21: support
m22: BDD membrane
m31: support
m32: BDD membrane
m41: support
m42: BDD membrane
L: electrode length
W: electrode width (flow channel width)
D: inter-electrode distance (gap)

## Claims

1. An electroporation device that applies an electric field to a suspension containing a biologically derived material and a bioactive substance using an electrode pair to introduce the bioactive substance into the biologically derived material,
wherein each of electrodes of the electrode pair has a boron-doped diamond membrane on a surface portion in contact with the suspension.

2. The electroporation device according to claim 1,
wherein a boron content of the boron-doped diamond membrane is 100 to 10,000 ppm.

3. The electroporation device according to claim 1,
wherein a resistivity of the boron-doped diamond membrane is 1 × 10⁻³ to 1 × 10⁻¹ Ω·cm.

4. The electroporation device according to claim 1,
wherein a membrane thickness of the boron-doped diamond membrane is 2 to 20 µm.

5. The electroporation device according to claim 1,
wherein an inter-electrode distance of the electrode pair is 1 to 10 mm.

6. The electroporation device according to claim 1,
wherein each of the electrodes includes a support and the boron-doped diamond membrane covering the support.

7. The electroporation device according to claim 6,
wherein a thickness of the support is 0.5 to 10 mm.

8. The electroporation device according to claim 6,
wherein the support is a metal substrate.

9. The electroporation device according to claim 8,
wherein a metal material of the metal substrate includes at least one selected from the group consisting of Mo, Nb, W, and Ti.

10. The electroporation device according to claim 1,
wherein the boron-doped diamond membrane is provided not only on the surface portion in contact with the suspension, but also on a portion that is equal to or more than half or 0.5 mm or more of a side surface portion of the electrode in a thickness direction or on an entire surface of the side surface portion continuously from the surface portion.

11. The electroporation device according to claim 1,
wherein a cooling mechanism that cools each of the electrodes is provided.

12. The electroporation device according to any one of claims 1 to 11 that is used for repeated batch electroporation,
wherein a container for accommodating the suspension is provided, and
the electrode pair is installed on an inner surface of the container so that the boron-doped diamond membranes of the electrodes face each other.

13. The electroporation device according to any one of claims 1 to 11 that is used for flow electroporation,
wherein a flow channel for allowing the suspension to flow is provided, and
the electrode pair is installed with the flow channel interposed therebetween so that the boron-doped diamond membranes of the electrodes face each other.
